# EUROPEAN PATENT SPECIFICATION

(11) **EP 4 429 535 B1**
(45) Date of publication and mention of the grant of the patent: **23.09.2026**
(21) Application number: 22821575.2
(22) Date of filing: 08.11.2022
(51) Int. Cl.: A61B 5/00, G06F 3/00

(54) **COMPUTER SYSTEM AND COMPUTER-IMPLEMENTED METHOD**
COMPUTERSYSTEM UND COMPUTERIMPLEMENTIERTES VERFAHREN
SYSTÈME INFORMATIQUE ET PROCÉDÉ MIS EN OEUVRE PAR ORDINATEUR

(30) Priority: 08.11.2021 GB 202116035; 22.03.2022 GB 202203990
(43) Date of publication of application: 18.09.2024
(73) Proprietor: X-System Limited, Whiteley, Hampshire PO15 7FX (GB)
(72) Inventor: OSBORNE, Ruaraidh, Whiteley Hampshire PO15 7FX (GB); WALTON, Jonathan, Whiteley Hampshire PO15 7FX (GB); OSBORNE, Nigel, Whiteley Hampshire PO15 7FX (GB)
(74) Representative: Langley, Peter James
(86) International application number: PCT/GB2022/052825
(87) International publication number: WO 2023/079318

(56) References cited:
- WO-A1-96/24906
- AU-A4- 2021 101 097
- US-A1- 2017 326 330
- US-A1- 2020 286 505
- FRANÇOIS B VIALATTE ET AL: "Sparse Bump Sonification: A New Tool for Multichannel EEG Diagnosis of Mental Disorders; Application to the Detection of the Early Stage of Alzheimer's Disease", 1 January 2006, NEURAL INFORMATION PROCESSING LECTURE NOTES IN COMPUTER SCIENCE;;LNCS, SPRINGER, BERLIN, DE, PAGE(S) 92 - 101, ISBN: 978-3-540-46484-6, XP019046646

## Description

### BACKGROUND OF THE INVENTION

### 1. Field of the Invention

The field of the invention relates to audio-based methods of treatment of neurological conditions in humans, and to computer systems and computer-implemented methods used in selecting audio suitable for use in audio-based methods of treatment of neurological conditions in humans.

### 2. Technical Background

600,000 people in the UK suffer from epilepsy, in which 200,000 cases are considered to be 'intractable'. The equivalent figures for the US are 3.5 million and 1 million people, respectively. Globally, approximately 1% of the population suffer from epilepsy. Current treatments for epilepsy range from medication, which may be permanent or temporary, to vagus nerve stimulation and brain surgery. Senior neurologists would welcome a new treatment, and related apparatus, that is highly effective, without damaging side-effects, and that is tailored to the individual. Senior neurologists would also welcome new treatments, and related apparatus, for other neurological conditions, that are highly effective, without damaging side-effects, and that are tailored to the individual.

### 3. Discussion of Related Art

WO2012168740A1, US9736603B2, US10587967B2 and EP2729931B1 disclose a method and system for analysing audio (eg. music) tracks. A predictive model of the neuro-physiological functioning and response to sounds by one or more of the human lower cortical, limbic and subcortical regions in the brain is described. Sounds are analysed so that appropriate sounds can be selected and played to a listener in order to stimulate and/or manipulate neuro-physiological arousal in that listener. The method and system are particularly applicable to applications harnessing a biofeedback resource.

EP2729931B1 discloses a computer-implemented method for analysing audio tracks for playback to a human subject according to a preselected desired arousal state of the human subject, wherein the arousal state of the human subject is indicated by galvanic skin conductance or by heart rate, comprising the steps of:
(i) storing a set of individual audio tracks operable for selection for playback;
(ii) predicting a neuro-physiological excitement response to the individual audio tracks according to a neuro-physiological model of the functioning and response of one or more of the human lower cortical, limbic and subcortical regions in the brain to sounds; and in which values of rhythmicity, inharmonicity and turbulence, of tracks, are automatically determined using signal processing techniques, and further comprising the step of combining the values of rhythmicity, inharmonicity and turbulence to yield a predictive value of excitement or arousal of the human subject, wherein for each track, turbulence T equals dH/dt * P, where H is harmonicity, P is the energy present during peaks of volume of the track, and t is time;
(iii) receiving a selected desired arousal state of the human subject;
(iv) selecting audio tracks according to the predictive value of neuro-physiological excitement response to the individual music tracks, and according to the selected desired arousal state of the human subject; and
(v) playing the selected audio tracks.

The patent applications US 2017/326330 A1 (BULAJ ET AL, 2017-11-16), US 2020/286505 A1 (OSBORNE ET AL, 2020-09-10), AU 2021 101 097 A4 (AMRITA ET AL, 2021-04-29), and WO 96/24906 A1 (JORDAN, 1996-08-15) are pertinent to understand the background of the invention. The scientific article titled "Sparse Bump Sonification: A New Tool for Multichannel EEG Diagnosis of Mental Disorders; Application to the Detection of the Early Stage of Alzheimer's Disease" (VIALATTE ET AL, 2006-01-01) is pertinent for the understanding of the invention.

### SUMMARY OF THE INVENTION

According to a first aspect of the invention, there is provided a computer system according to claim 1, the computer system including music audio files including respective music file audio data, the computer system configured to:
(i) receive a file of electroencephalogram (EEG) data comprising EEG data of healthy brain behaviour of a human subject;
(ii) audify the EEG data of healthy brain behaviour of the human subject;
(iii) analyse the audified EEG data according to a neuro-physiological model of the principal areas and networks of the human brain involved in processing music, to produce analysis data of the audified EEG data;
(iv) analyse the music file audio data according to the neuro-physiological model of the principal areas and networks of the human brain involved in processing music, to produce analysis data of the music file audio data;
(v) compare the analysis data of the audified EEG data with the analysis data of the music file audio data, to match the analysis data of the audified EEG data with matched analysis data of the music file audio data, to produce a playlist of matched music audio files corresponding to the matched music file audio data, wherein the matched music audio files are suitable to entrain healthy brain behaviour in the human subject; and
(vi) store the playlist of matched music audio files.

An advantage is that the stored playlist of matched music audio files can be played to entrain healthy brain behaviour in the human subject. An advantage is that the stored playlist of matched music audio files can be played to provide treatment of a neurological condition in the human subject, e.g. epilepsy, e.g. epilepsy in the case of a child, e.g. intractable epilepsy, e.g. intractable epilepsy in the case of a child.

The computer system (e.g. an audio playback device) may include a speaker or headphones or a sound reproduction device, wherein the computer system is further configured to play the playlist of matched music audio files to the human subject, including outputting played matched music audio files to the speaker or to the headphones or to the sound reproduction device. An advantage is that the playlist can be played to entrain healthy brain behaviour in the human subject. Headphones include the example of earbuds. A sound reproduction device includes the example of directional speakers. An advantage is that the playlist can be played to provide treatment of a neurological condition in the human subject, e.g. epilepsy, e.g. epilepsy in the case of a child, e.g. intractable epilepsy, e.g. intractable epilepsy in the case of a child.

The computer system may be one wherein audifying the EEG data comprises:
(a) sampling the EEG data at a rate in the range of 100 Hz to 2.5kHz;
(b) extracting sinusoidal waves with time varying frequency from the sampled EEG data from at least three frequency bands in the range of 0.8 to 40 Hz;
(c) upsampling the extracted sinusoidal waves with time varying frequency to a rate in the range of 10 kHz to 100kHz;
(d) constructing signals for the upsampled extracted sinusoidal waves with time varying frequency by scaling the upsampled extracted sinusoidal waves with time varying frequency in the range of 5 to 9 octaves, to provide audified EEG data.

An advantage is that the audified EEG data is suitably prepared for analysis according to a neuro-physiological model of the principal areas and networks of the human brain involved in processing music, to produce related analysis data of the audified EEG data.

The computer system may be one wherein in (a) the EEG data is sampled at a rate in the range of 200 Hz to 1 kHz. The computer system may be one wherein in (a) the EEG data is sampled at a rate of 500 Hz. An advantage is that the audified EEG data is suitably prepared for analysis according to a neuro-physiological model of the principal areas and networks of the human brain involved in processing music.

The computer system may be one wherein (a) includes subtracting a 3rd order polynomial fit to remove trends which can skew time-frequency representation (TFR) and then the signal is low-pass filtered at the Nyquist frequency.

The computer system may be one wherein in (b) there are three frequency bands. The computer system may be one wherein in (b) there are three frequency bands which are 0.8-3.5Hz, 3.5-12Hz, and 12-40Hz. An advantage is that the audified EEG data is suitably prepared for analysis according to a neuro-physiological model of the principal areas and networks of the human brain involved in processing music.

The computer system may be one wherein in (b) the analysis is performed using time-frequency representations (TFRs).

The computer system may be one wherein in (b) ridge extraction is used. In an example, ridge extraction is an algorithm which is part of MODA. The computer system may be one wherein in (b) extracting sinusoidal waves with time varying frequency from the sampled EEG data is performed using Multiscale oscillatory dynamics analysis (MODA). The computer system may be one wherein the sinusoidal waves with time varying frequency are extracted from the sampled EEG data using the algorithm 'ridge extraction', which is part of the open source package 'Multiscale oscillatory dynamics analysis (MODA). The computer system may be one wherein the time varying frequencies of the sinusoidal waves are the dominant frequencies within the chosen frequency band, which may be 0.8-3.5Hz, etc. An advantage is that the audified EEG data is suitably prepared for analysis according to a neuro-physiological model of the principal areas and networks of the human brain involved in processing music.

The computer system may be one wherein in (c ) the extracted sinusoidal waves with time varying frequency are upsampled to a rate in the range from 20 kHz to 80 kHz. The computer system may be one wherein in (c ) the extracted sinusoidal waves with time varying frequency are upsampled to a rate of 44.5 kHz. The computer system may be one wherein in (c ) the waves are upsampled by inserting the appropriate amount of samples along a straight line connecting each pair of old sample points. The computer system may be one wherein in (d), the upsampled extracted sinusoidal waves with time varying frequency are scaled by 5 octaves, or by 6 octaves, or by 7 octaves, or by 8 octaves, or by 9 octaves. The computer system may be one wherein in (d), a factor of 2 to the power of the number of octaves to be scaled by is inserted into a ridge reconstruction equation, e.g. Eq. (2). An advantage is that the audified EEG data is suitably prepared for analysis according to a neuro-physiological model of the principal areas and networks of the human brain involved in processing music.

The computer system may be one wherein in parts (iii) and (iv), the analysis is performed by analysing for volume, turbulence, sharpness, rhythmicity, and harmonicity H. The computer system may be one wherein the analysis for volume, turbulence, sharpness, rhythmicity, and harmonicity H is performed using signal processing techniques. The computer system may be one wherein harmonicity is analysed by analysing for chroma and pitch height, as well as for fundamentals and spectra. The computer system may be one wherein analysing for harmonicity includes analysing for linear harmonic cost. The computer system may be one wherein rhythmicity analysis includes detecting power, salience and density of periodic spectral turbulence. The computer system may be one wherein turbulence is dH/dt * P, where P is the energy present during peaks of volume of the data, and t is time. The computer system may be one wherein in parts (iii) and (iv), the analysis is performed by using X-System. An advantage is that the audified EEG data is suitably prepared for analysis according to a neuro-physiological model of the principal areas and networks of the human brain involved in processing music.

The computer system may be one wherein the file of electroencephalogram (EEG) data comprising EEG data of healthy brain behaviour of a human subject is in the range of 1 minute to 100 minutes in duration. The computer system may be one wherein the file of electroencephalogram (EEG) data comprising EEG data of healthy brain behaviour of a human subject is in the range of 3 minutes to 30 minutes in duration. The computer system may be one wherein the file of electroencephalogram (EEG) data comprising EEG data of healthy brain behaviour of a human subject is 10 minutes in duration.

The computer system may be one wherein the file of electroencephalogram (EEG) data comprising EEG data of healthy brain behaviour of a human subject begins with EEG data corresponding to wakefulness, then continues with EEG data corresponding to sleep. An advantage is that the playlist can be played to take the human subject from wakefulness, to sleep.

The computer system may be one wherein the file of electroencephalogram (EEG) data comprising EEG data of healthy brain behaviour of a human subject includes only EEG data corresponding to sleep. An advantage is that the playlist can be played to maintain the human subject in a state of sleep.

The computer system may be one wherein the playlist is 1 to 12 hours in duration. The computer system may be one wherein the playlist is 6 to 10 hours in duration. The computer system may be one wherein the playlist is 9 hours in duration. The computer system may be one wherein the playlist includes music composed by Mozart.

The computer system may be one wherein the playlist is processed by generation of a playlist audio data file.

The computer system may be one wherein the playlist audio data file is processed by silence being trimmed from the start and the end of the playlist audio data file. The computer system may be one wherein the playlist audio data file is processed by amplitude normalisation to a peak of -0.1 dB. The computer system may be one wherein the playlist audio data file is processed by cross-fading of 0.5-30 seconds at start and end of each track, or 5-10 seconds at start and end of each track. The computer system may be one wherein the playlist audio data file is processed by gain being reduced in the frequency range 250-2000 Hz for tracks containing solo female or male vocals or prominent solo or ensemble instruments. The computer system may be one wherein the playlist audio data file is processed by compression being applied with a large ratio and low threshold, in order to remove large changes in dynamics which risk waking a patient, particularly between sleep cycles. The computer system may be one wherein the playlist audio data file is exported as a single file. The computer system may be one wherein the playlist audio data file is exported as a single file MP3, WAV, AIFF, OGG, .AAC, WMA or other audio format files, 44.1kHz, or 48 / 96 kHz. The computer system may be one wherein the playlist audio data file is processed by tags being added to the file for identification and cross-platform compatibility. The computer system may be one wherein the playlist audio data file is processed by ID3v2 or ID3v1 tags being added to the file for identification and cross-platform compatibility. An advantage is that the stored playlist of matched music audio files can be played to entrain healthy brain behaviour in the human subject. An advantage is that the playlist audio data file can be played to provide improved treatment of a neurological condition in the human subject, e.g. epilepsy, e.g. epilepsy in the case of a child, e.g. intractable epilepsy, e.g. intractable epilepsy in the case of a child, including the case where the human subject is asleep. An advantage is that the playlist audio data file is less likely to awaken the human subject during treatment.

According to a second aspect of the invention, there is provided a computer-implemented method, as defined in Claim 15, for generating a playlist of music audio files suitable to provide healthy brain behaviour in a human subject, the method including the steps of:
(i) receiving a file of electroencephalogram (EEG) data comprising EEG data of healthy brain behaviour of a human subject;
(ii) audifying the EEG data of healthy brain behaviour of the human subject;
(iii) analysing the audified EEG data according to a neuro-physiological model of the principal areas and networks of the human brain involved in processing music, to produce analysis data of the audified EEG data;
(iv) accessing music audio files including respective music file audio data;
(v) analysing the music file audio data of the music audio files according to the neuro-physiological model of the principal areas and networks of the human brain involved in processing music, to produce analysis data of the music file audio data;
(vi) comparing the analysis data of the audified EEG data with the analysis data of the music file audio data, to match the analysis data of the audified EEG data with matched analysis data of the music file audio data, to produce a playlist of matched music audio files corresponding to the matched music file audio data, wherein the matched music audio files are suitable to entrain healthy brain behaviour in the human subject; and
(vii) storing the playlist of matched music audio files.

An advantage is that the stored playlist of matched music audio files can be played to entrain healthy brain behaviour in the human subject. An advantage is that the stored playlist of matched music audio files can be played to provide treatment of a neurological condition in the human subject, e.g. epilepsy, e.g. epilepsy in the case of a child, e.g. intractable epilepsy, e.g. intractable epilepsy in the case of a child.

The method may be one wherein audifying the EEG data comprises:
(a) sampling the EEG data at a rate in the range of 100 Hz to 2.5kHz;
(b) extracting sinusoidal waves with time varying frequency from the sampled EEG data from at least three frequency bands in the range of 0.8 to 40 Hz;
(c) upsampling the extracted sinusoidal waves with time varying frequency to a rate in the range of 10 kHz to 100kHz;
(d) constructing signals for the upsampled extracted sinusoidal waves with time varying frequency by scaling the upsampled extracted sinusoidal waves with time varying frequency in the range of 5 to 9 octaves, to provide audified EEG data.

An advantage is that the audified EEG data is suitably prepared for analysis according to a neuro-physiological model of the principal areas and networks of the human brain involved in processing music, to produce related analysis data of the audified EEG data.

The method may be one wherein in (a) the EEG data is sampled at a rate in the range of 200 Hz to 1 kHz. The method may be one wherein in (a) the EEG data is sampled at a rate of 500 Hz. An advantage is that the audified EEG data is suitably prepared for analysis according to a neuro-physiological model of the principal areas and networks of the human brain involved in processing music.

The method may be one wherein (a) includes subtracting a 3rd order polynomial fit to remove trends which can skew time-frequency representation (TFR) and then the signal is low-pass filtered at the Nyquist frequency.

The method may be one wherein in (b) there are three frequency bands. The method may be one wherein in (b) there are three frequency bands which are 0.8-3.5Hz, 3.5-12Hz, and 12-40Hz. An advantage is that the audified EEG data is suitably prepared for analysis according to a neuro-physiological model of the principal areas and networks of the human brain involved in processing music.

The method may be one wherein in (b) the analysis is performed using time-frequency representations (TFRs).

The method may be one wherein in (b) ridge extraction is used. In an example, ridge extraction is an algorithm which is part of MODA. The method may be one wherein in (b) extracting sinusoidal waves with time varying frequency from the sampled EEG data is performed using Multiscale oscillatory dynamics analysis (MODA). The method may be one wherein the sinusoidal waves with time varying frequency are extracted from the sampled EEG data using the algorithm 'ridge extraction', which is part of the open source package 'Multiscale oscillatory dynamics analysis (MODA). The method may be one wherein the time varying frequencies of the sinusoidal waves are the dominant frequencies within the chosen frequency band, which may be 0.8-3.5Hz, etc. An advantage is that the audified EEG data is suitably prepared for analysis according to a neuro-physiological model of the principal areas and networks of the human brain involved in processing music.

The method may be one wherein in (c ) the extracted sinusoidal waves with time varying frequency are upsampled to a rate in the range from 20 kHz to 80 kHz. The method may be one wherein in (c ) the extracted sinusoidal waves with time varying frequency are upsampled to a rate of 44.5 kHz. The method may be one wherein in (c ) the waves are upsampled by inserting the appropriate amount of samples along a straight line connecting each pair of old sample points. The method may be one wherein in (d), the upsampled extracted sinusoidal waves with time varying frequency are scaled by 5 octaves, or by 6 octaves, or by 7 octaves, or by 8 octaves, or by 9 octaves. The method may be one wherein in (d), a factor of 2 to the power of the number of octaves to be scaled by is inserted into a ridge reconstruction equation, e.g. Eq. (2). An advantage is that the audified EEG data is suitably prepared for analysis according to a neuro-physiological model of the principal areas and networks of the human brain involved in processing music.

The method may be one wherein in steps (iii) and (v), the analysis is performed by analysing for volume, turbulence, sharpness, rhythmicity, and harmonicity H. The method may be one wherein the analysis for volume, turbulence, sharpness, rhythmicity, and harmonicity H is performed using signal processing techniques. The method may be one wherein harmonicity is analysed by analysing for chroma and pitch height, as well as for fundamentals and spectra. The method may be one wherein analysing for harmonicity includes analysing for linear harmonic cost. The method may be one wherein rhythmicity analysis includes detecting power, salience and density of periodic spectral turbulence. The method may be one wherein turbulence is dH/dt * P, where P is the energy present during peaks of volume of the data, and t is time. The method may be one wherein in steps (iii) and (v), the analysis is performed by using X-System. An advantage is that the audified EEG data is suitably prepared for analysis according to a neuro-physiological model of the principal areas and networks of the human brain involved in processing music.

The method may be one wherein the file of electroencephalogram (EEG) data comprising EEG data of healthy brain behaviour of a human subject is in the range of 1 minute to 100 minutes in duration. The method may be one wherein the file of electroencephalogram (EEG) data comprising EEG data of healthy brain behaviour of a human subject is in the range of 3 minutes to 30 minutes in duration. The method may be one wherein the file of electroencephalogram (EEG) data comprising EEG data of healthy brain behaviour of a human subject is 10 minutes in duration.

The method may be one wherein the file of electroencephalogram (EEG) data comprising EEG data of healthy brain behaviour of a human subject begins with EEG data corresponding to wakefulness, then continues with EEG data corresponding to sleep. An advantage is that the playlist can be played to take the human subject from wakefulness, to sleep.

The method may be one wherein the file of electroencephalogram (EEG) data comprising EEG data of healthy brain behaviour of a human subject includes only EEG data corresponding to sleep. An advantage is that the playlist can be played to maintain the human subject in a state of sleep.

The method may be one wherein the playlist is 1 to 12 hours in duration. The method may be one wherein the playlist is 6 to 10 hours in duration. The method may be one wherein the playlist is 9 hours in duration. The method may be one wherein the playlist includes music composed by Mozart.

The method may be one wherein the playlist is processed by generation of a playlist audio data file.

The method may be one wherein the playlist audio data file is processed by silence being trimmed from the start and the end of the playlist audio data file. The method may be one wherein the playlist audio data file is processed by amplitude normalisation to a peak of -0.1 dB. The method may be one wherein the playlist audio data file is processed by cross-fading of 0.5-30 seconds at start and end of each track, or 5-10 seconds at start and end of each track. The method may be one wherein the playlist audio data file is processed by gain being reduced in the frequency range 250-2000 Hz for tracks containing solo female or male vocals or prominent solo or ensemble instruments. The method may be one wherein the playlist audio data file is processed by compression being applied with a large ratio and low threshold, in order to remove large changes in dynamics which risk waking a patient, particularly between sleep cycles. The method may be one wherein the playlist audio data file is exported as a single file. The method may be one wherein the playlist audio data file is exported as a single file MP3, WAV, AIFF, OGG, .AAC, WMA or other audio format files, 44.1kHz or 48 / 96 kHz. The method may be one wherein the playlist audio data file is processed by tags being added to the file for identification and cross-platform compatibility. The method may be one wherein the playlist audio data file is processed by ID3v2 or ID3v1 tags being added to the file for identification and cross-platform compatibility. An advantage is that the stored playlist of matched music audio files can be played to entrain healthy brain behaviour in the human subject. An advantage is that the playlist audio data file can be played to provide improved treatment of a neurological condition in the human subject, e.g. epilepsy, e.g. epilepsy in the case of a child, e.g. intractable epilepsy, e.g. intractable epilepsy in the case of a child, including the case where the human subject is asleep. An advantage is that the playlist audio data file is less likely to awaken the human subject during treatment.

According to a third aspect of the disclosure, which is not part of the claimed invention, there is provided a playlist of matched music audio files generated by the computer-implemented method of any aspect of the second aspect of the invention.

According to a fourth aspect of the disclosure, which is not part of the claimed invention, there is provided a method of treatment of a human subject, the method including the step of playing a playlist of matched music audio files, generated by the computer-implemented method of any aspect of the second aspect of the invention, to the human subject, to entrain healthy brain behaviour in the human subject, including musical entrainment of brain activity.

An advantage is that the playlist can be played to entrain healthy brain behaviour in the human subject. An advantage is that the playlist can be played to provide treatment of a neurological condition in the human subject, e.g. epilepsy, e.g. epilepsy in the case of a child, e.g. intractable epilepsy, e.g. intractable epilepsy in the case of a child.

The method may include providing a reduction in spikes (e.g. inter-ictal epileptiform discharges) in brain activity.

The method may include improving the quality of sleep of the human subject.

The method may be one wherein the human subject is a child.

The method may be one wherein the human subject is an adult.

The method may be one wherein the method of treatment includes treatment of epilepsy.

The method may be one wherein the method of treatment includes treatment of general anxiety, or panic disorder, or Post-traumatic stress disorder (PTSD), or sleep disorders, or chronic pain, or depression, or pre-operative anxiety, or mental state during specific medical procedures, or post-operative pain management.

The method may be one wherein the method of treatment includes treatment of a severely psychotic patient in a mental institution.

The method may be one wherein the method of treatment includes treatment of rare epilepsies and/or movement disorders which have not responded to available medications.

The method may be one wherein the method of treatment includes providing mood management and/or general mental wellbeing.

According to a fifth aspect of the disclosure, which is not part of the claimed invention, there is provided a computer system (e.g. an audio playback device) including a speaker or headphones or a sound reproduction device, the computer system including a playlist of matched music audio files according to the third aspect of the invention, the computer system configured to play the playlist of matched music audio files, including outputting the played matched music audio files to the speaker or to the headphones or to the sound reproduction device.

An advantage is that the computer system can play the playlist to entrain healthy brain behaviour in the human subject. An advantage is that the computer system can play the playlist to provide treatment of a neurological condition in the human subject, e.g. epilepsy, e.g. epilepsy in the case of a child, e.g. intractable epilepsy, e.g. intractable epilepsy in the case of a child.

The computer system may be configured to perform a method of any aspect of the fourth aspect of the invention.

### BRIEF DESCRIPTION OF THE FIGURES

Aspects of the disclosure will now be described, by way of example(s), with reference to the following Figures, in which:

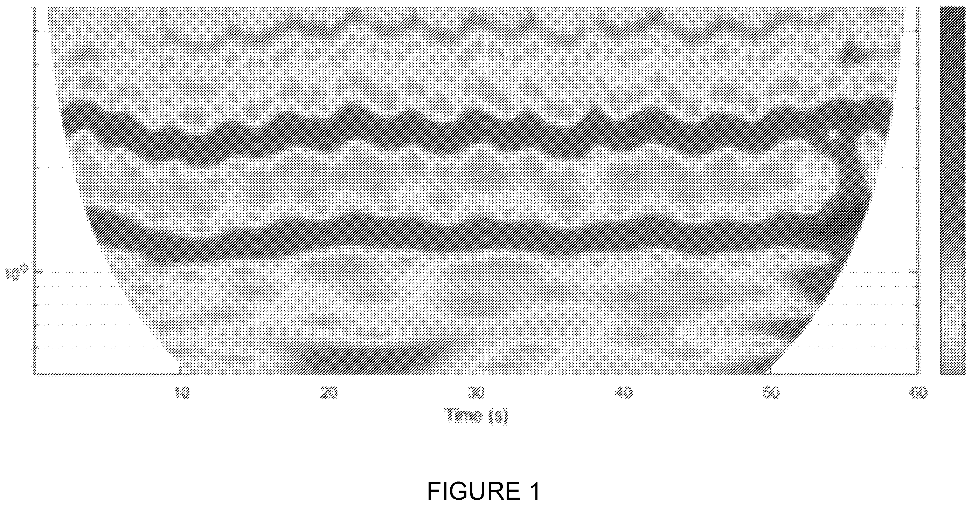

**Figure 1** shows an example of a time frequency response (TFR) of 1 minute of electrocardiogram (ECG) signal. Frequency is plotted logarithmically on the y-axis and the darkness indicates the square root of the power of that frequency within the signal. The quasi-steady resting heart rate can be seen to evolve in time as the 'ridge' lying just above 1Hz; above that we have three visible harmonics with the first and the third being of higher amplitude. The effects of the uncertainty principle and logarithmic scaling can be seen in the refined time resolution of the first harmonic over the fundamental and the appearance of 'vertical lines' in the upper harmonics. These vertical lines are components over-resolved in time while the fundamental frequency lacks the time dependent details due to over resolution in frequency.

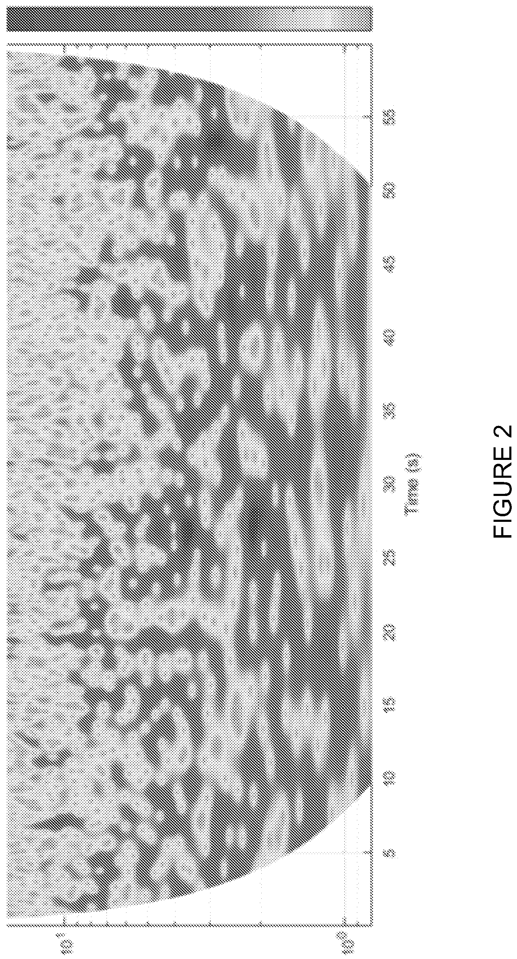

**Figure 2** shows an example of electroencephalogram (EEG) data from a minute of rapid eye movement (REM) sleep from patient 'x' while listening to music composed by Debussy. TFRs of EEG data are much more complicated than TFRs of ECG data. Here resolution has been chosen to give clarity to the theta/alpha region, so lower frequencies are overly resolved in frequencies and higher frequencies are over resolved in time (i.e. there is a tendency towards horizontal and vertical lines respectively).

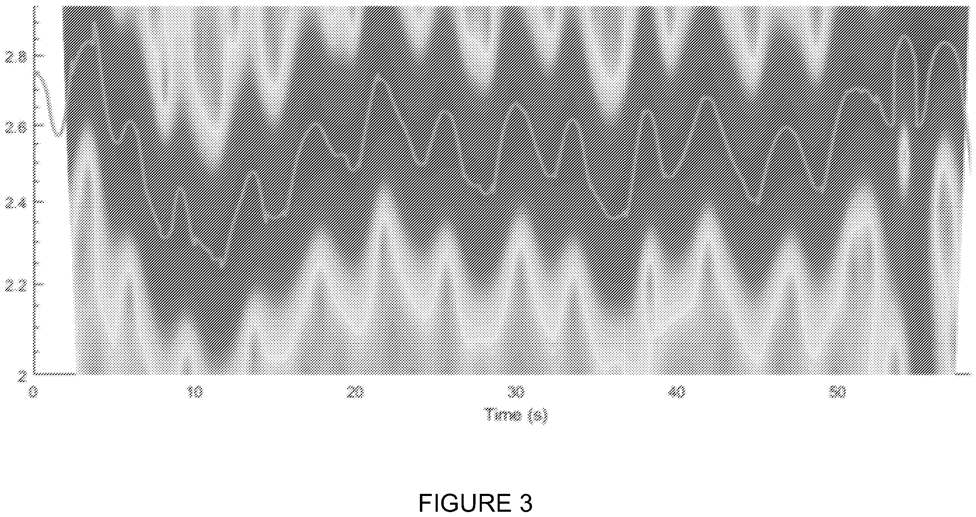

**Figure 3** shows a straightforward example of Ridge extraction. The well-defined first harmonic ridge from **Figure 1** is extracted as the wavy line in this figure. MODA extracts the instantaneous frequency, amplitude (power) and phase at each sample point on the ridge.

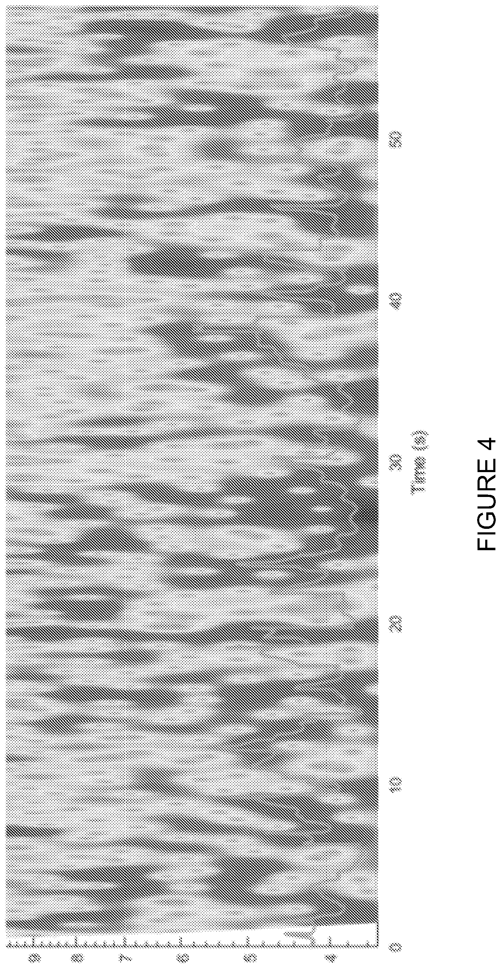

**Figure 4** shows an example in which ridges are much less well defined in EEG data than in ECG data. This ridge extract shown here, from the 3.5-12 Hz region of **Figure 2****,** is mostly confined below 5Hz (see the wavy line), corresponding to delta activity.

### DETAILED DESCRIPTION

### Musical entrainment of brain activity in people (e.g. in children), e.g. with epilepsy, e.g. using X-system and MODA

What follows includes a description of musical treatment of epilepsy in people. In particular, sections describing the selection and prescription of the music are presented.

### Entrainment

There have been a number of studies into the effects of music on epilepsy, in particular related to the so-called "Mozart effect", in which listening to Mozart's music is said to decrease seizures in children with epilepsy. Underlying all of these studies is the premise that music may affect or even "entrain" electrical brain activity. There is a substantial literature, extending over almost half a century, examining this phenomenon, including effects of different kinds of music, different tempi, and even imagined rhythmic patterns (e.g. Rafiee et al. (2021); Okawa et al. (2017); Ramos and Corsi-cabrera (1989); Breitling et al. (1987)). Some of the work presented here is based on the assumption that music that closely resembles the time and frequency profiles of healthy electrical brain activity of individual patients may be used to entrain such activity in the same patients. In other words, it is worth investigating whether Audifications of electroencephalogram (EEG) data from healthy brain activity of children with epilepsy may be used to help regulate their brains in a healthy way, including reducing spikes (e.g. inter-ictal epileptiform discharges) and improving quality of sleep.

### Summary of Methods

In an example, the challenge of identifying music that may entrain healthy brain behaviour was tackled in four stages. First, healthy sections of EEG data roughly 10 minutes long were recommended for each patient by a professional neurologist with over 30 years of experience reading EEG data of patients with epilepsy. Then, that healthy EEG data was audified as described in the section Audification, producing as accurate an audio representation of healthy brain activity as possible. The audification was then compared to existing musical repertoire in certain genres using X-system as described in the section X-System Curation to produce candidate playlists containing pieces that matched the audification in terms of X-System parameters (see below, for example). The audio files in the playlist were then additionally treated to prevent disturbances in the night as described in the section Audio Processing. We also describe in the section Patient Delivery how the playlists were administered to the children. Before explaining our main methods, a short summary of X-system and MODA are given below.

### Background about X-system

X-System, which has been developed over the last decade for both medical and musical purposes, models the principal areas and networks of the brain involved in processing music. Brain stem responses to sounds of primal evolutionary/survival value - for example startling, rapidly approaching or very high sounds (Sivaramakrishnan et al. (2004), Osborne (2009), Erlich et al. (2013), Frankland et al. (1997), Panksepp (2003)) - are modelled by volume, turbulence and sharpness algorithms, as are related ascending pathways by way of the inferior colliculus to the amygdala (JORIS et al. (2004), Heldt and Falls (2003), Marsh et al. (2002)). The responses of the basal ganglia, cerebellum, premotor and motor cortex (Sacks (2007), Panksepp (2004)) are modelled by rhythmicity algorithms, detecting the power, salience and density of periodic spectral turbulence (Osborne (2009)); this forms part of a complex loop with processing and retention of patterns in the auditory cortex, including the right anterior secondary cortex (Peretz (2001), Penhune et al. (1999), Peretz and Kolinsky (1993)) modelled by autocorrelation and related to tempo and metrical structures. There are algorithms that as far as possible replicate basic pitch detection in the auditory brain stem as well as more complex modelling of Heschl's gyrus. Here, chroma and pitch height are detected (Griffiths et al. (1998), Warren et al. (2003)), as well as fundamentals and spectra (Schneider et al. (2005), Menon et al. (2002)). Important outputs of these models are indicators of levels of harmonicity (how close the spectrum is to the harmonic series) and the resulting activation of limbic and paralimbic systems (Peretz et al. (2010), McDermott et al. (2010) Koelsch et al. (2007), Stein et al. (2007), Baumgartner et al. (2006), Eldar et al. (2007), Blood and Zatorre (2001)). These are measures of "vertical" harmonicity, but in pathways to emotional centres, for example the amygdala, "linear" harmonicity, or how notes and chords follow one another, is also significant (e.g. Koelsch et al. (2008)), and is modelled by a linear harmonic cost algorithm. X-System may not only predict autonomic effects of music on electrical activity in the brain, but also identify, through its wealth of models and parameters, and through use of audifications of EEG data to search for appropriate tracks, music that most resembles the healthy brain activity of patients.

### Background about MODA

Multiscale oscillatory dynamics analysis (MODA), is an open source software package for analysing time-series data using wavelets developed by members of the Nonlinear and Biomedical Physics group at the University of Lancaster and the Nonlinear Dynamics and Synergetic Group at the Faculty of Electrical Engineering in the University of Ljubljana. Wavelets are commonly used in time series analysis to provide time-frequency representations (TFRs) of a signal much like the more widely known windowed Fourier transform (WFT). In both transforms one always has to contend with the Heisenberg uncertainty principle, or bandwidth theorem, restricting the accuracy of one's simultaneous knowledge of time and frequency in a signal. However, while WFTs are restricted to fixed time and frequency resolution at all scales, windowed transforms (WTs) have a logarithmically scaled frequency resolution allowing frequency-skewed resolution at low frequencies and prone-to-error frequencies while offering time-skewed accuracy at easier-to-detect higher frequencies (see Iatsenko et al. (2015)).

### Time-Frequency Representations of Signals

TFRs have a plethora of uses within the medical sciences (e.g Unser and Aldroubi (1996)) from medical imaging to automatic inter-ictal epileptiform spike detection in EEG data (İnan Güler and Übeyli (2005) and Faust et al. (2015) for example). TFRs tell you what frequencies are present in a signal and how much of the signal's power is carried by that frequency for each moment in time the signal is sampled at. See **Figure 1****,** for example. In many physiological systems, in particular cardiovascular and respiratory systems (see Iatsenko et al. (2013) and Ticcinelli et al. (2017) for instance), the measurable biophysical signal includes, or consists of, one or more oscillators with a well defined frequency which may slowly change in time. In the most transparent example, the periodic gap between QRS (Pérez Riera et al. (2016)) peaks in an electrocardiogram (ECG) signal appear as an almost horizontal line in time-frequency space usually between 0.6Hz and 2Hz. The QRS complex is the combination of three of the graphical deflections seen on a typical electrocardiogram (ECG). It is usually the central and most visually obvious part of the tracing. It corresponds to the depolarization of the right and left ventricles of the heart and contraction of the large ventricular muscles. In adults, the QRS complex normally lasts 80 to 100 ms; in children it may be shorter. The Q, R, and S waves occur in rapid succession, do not all appear in all leads, and reflect a single event and thus are usually considered together. A Q wave is any downward deflection immediately following the P wave. An R wave follows as an upward deflection, and the S wave is any downward deflection after the R wave. The T wave follows the S wave, and in some cases, an additional U wave follows the T wave. To measure the QRS interval, start at the end of the PR interval (or beginning of the Q wave) to the end of the S wave. Normally this interval is 0.08 to 0.10 seconds. When the duration is longer it is considered a wide QRS complex.

### Ridge Extraction

This leads us to the useful concept of a 'ridge' in a TFR. Within a certain frequency band, a ridge is a line connecting the dominant frequency in that band at each sample in time (Iatsenko et al. (2016)) (see **Figure 3** for example). One of MODA's analysis packages, 'ridge extraction', can extract ridges from complex signals as amplitude and phase pairs at each sample in time, allowing for the ridge to be 'reconstructed' as a sine wave with time dependent amplitude and frequency, acting as a filter on the original signal. Explicitly, for a signal sampled at *fₛ* Hz, at each time sample t (measured in seconds) ridge extractions retrieves the dominant frequency *f*(*t*), its amplitude within the wavelet decomposition (Iatsenko et al. (2015)) *A_{f}(t)* (where the physical power of that frequency is given by *A_{f}*² ), and the phase of that oscillating component *ϕ_{f}*(*t*) where $f \left(t\right) = {dϕ}_{f} \left(t\right) / dt = {f}_{s} \left({ϕ}_{f}\left(t\right)-{ϕ}_{f}\left(t-1/{f}_{s}\right)\right) .$

The signal can be filtered down to a single sinusoidal component with time varying frequency that follows the ridge using the ridge reconstruction equation $R \left(t\right) = {A}_{f} \left(t\right) cos {ϕ}_{f} \left(t\right) .$

In an example, this equation represents a key step in our audification process; by filtering down the signal to a single sinusoidal component with time varying frequency, instead of a bandpass filter, we can easily scale the frequency by 6, 7 or even 8 octaves, for example, without distorting the data in a serious way. In contrast, bandpass filtered signals pitched up by such an extreme amount using phase vocoders (Flanagan and Golden (1966)) exhibit serious phasing problems (Laroche and Dolson (1999)) which distorts the signal beyond recognition. The reason that a single sinusoidal component with time varying frequency is used is that these are not sine waves as such, but rather waveforms derived using a sine envelope.

In EEG signals, alpha, theta, delta, etc activity rarely appear as pure ridges (see **Figure 4** for example), however ridge extraction can offer a useful approximation for the activity. Certain activity in EEG data, even when viewed at high frequency resolution, appear as vertical lines indicating events in the brain triggering neuronal firings at multiple frequencies at once. However, within certain bands there is often an approximate ridge forming between regions of high power activity and regions with less power (see **Figure 4** for example).

### Audification

In an example, to produce audifications, the raw EEG data was sampled at a high rate of 500Hz in anticipation of its transformation into a music signal (>8000Hz) and it was recorded without any filters. The signal was then pre-processed in MODA, first a 3rd order polynomial fit was subtracted to remove trends which can skew the TFR and then the signal was low-pass filtered at the Nyquist frequency (half the sampling frequency) (see for example the supplementary section of Iatsenko et al. (2015). The strategy for audifying the EEG data started with extracting sinusoidal waves with time varying frequency from three frequency bands (0.8-3.5Hz, 3.5-12Hz, 12-40Hz) roughly corresponding to delta, theta/alpha and gamma activity respectively to act as three 'voices' for the audification. These waves were extracted using MODAs 'ridge extraction' protocol (see the section on MODA). Next, the waves were upsampled from 500Hz to 44.5kHz by inserting the appropriate amount of samples along a straight line connecting each pair of old sample points. While appearing quite artificial, this step is important (e.g. it is crucial) for allowing the waves to be interpreted by the ear as sound without speeding it up and the primitive linear interpolation of the new samples shouldn't interfere with the much lower frequency brain wave information. The final step was to reconstruct each ridge with its time varying frequency scaled by 7 octaves (2⁷) so that our three brain waves became three instruments with frequency ranges of 102.4-448 Hz, 448-1536 Hz and 1536-5120 Hz. The reconstruction was done simply by inserting a factor of 2⁷ into the argument of the cosine function in the ridge reconstruction equation, Equation (2).

### X-System Curation

In an example of X-System usage, the EEG audification track is uploaded and analysed, and a best-fit algorithm suggests tracks from the database which match X-System analysis of the EEG audification using the parameters described in the section Background about X-System. Appropriate genres are selected according to the cultural background and age of the patient. In the case of the pilot study, the following genres were chosen:
- Romantic
- Classical
- Baroque
- Minimalist
- World/Acoustic

In this first stage, tracks are curated manually on X-System and included or excluded based primarily on aesthetic factors (e.g. whether the track fits in the playlist in terms of instrumentation, genre and appropriateness for night-time listening). From these tracks, a single, curated night-time playlist is created with a duration of approximately nine hours, comprising or consisting of the sub-playlists taken from the genres above.

The first playlist begins with music with arousal values corresponding to heart rate during wakefulness, with subsequent tracks being of decreasing arousal value down to final tracks with arousal value corresponding to heart rate during sleep. For playlists 2, 3 and 4, which are intended to be listened to during sleep, tracks are separated by genre (and in the case of the classical music playlist, Mozart tracks are grouped together within that playlist in a single block).

### Audio Processing

In an example, following the completion of playlist generation and sequencing on X-System, master tracks are downloaded individually in playlist order and loaded into software for post-processing.

In the pilot study, the open-source Audacity programme was used for audio processing, and the open-source Kid3 programme for ID3 tag implementation.

During this example stage, further curation takes place based on technical considerations relating to sound quality, with excluded tracks being removed from the playlist. Audio system measurements are used to ensure a uniform listening experience across the night, taking into account recording fidelity and aspects of sound quality, such as amplitude, noise and psychoacoustic considerations relating to sudden changes in frequency and amplitude.

Once these tracks have been removed, the following processing takes place in this order:
1. Silence is trimmed from the front and back
2. Amplitude normalisation to a peak of -0.1 dB
3. Cross-fading of 0.5-30 seconds, or 5-10 seconds, at front and back of each track
4. Gain reduced in the frequency range 250-2000 Hz for tracks containing solo female or male vocals or prominent solo or ensemble instruments
5. Compression applied with a large ratio and low threshold, in order to remove large changes in dynamics which risk waking a patient, particularly between sleep cycles
6. Playlist is exported as a single file MP3, WAV, AIFF, OGG,. AAC, WMA or other audio format files, 44.1kHz or 48 / 96 kHz
7. ID3v2 or ID3v1 tags are added to the file for identification and cross-platform compatibility

### Patient Delivery

In an example, the following equipment is used for setting up and delivering the playlist to the patient.
- Decibel meter: ExaMobile Sound Meter v 1.2.6.233
- Speakers: Presonus E3.5 BT
- Media player: SanDisk MP3 Clip Sport GO 16 GB blue
- Audio cable: Adam Hall Cables 4 Star Series - Audio Cable REAN 3.5 mm Jack stereo to 2 x 6.3 mm Jack mono

The media player was empty with the exception of the playlist file.

Speakers were placed 86cm (+/- 3cm) from the centre of the pillow.

The decibel meter was calibrated to the ambient sound level in the room, and then, with the decibel meter directly in front of the speaker, playback volume was set to 46-50 dB. A physical mark was placed on the speaker to facilitate reproduction of this volume setting on subsequent nights.

### X-System music-based epilepsy treatment system

In an example, there are four steps to the process used in one implementation:
1. Neurologist takes an overnight EEG data of the patient and selects from this a period or periods of time during which brain activity is healthy.
2. X-System 'sonifies' this data and adapts it in order that our 'Innate Neuro-physiological Response to Music' (INRM) technology can be used to select music from the repertoire that will entrain further periods of healthy brain activity.
3. X-System technology is used to create a playlist that will first of all help the patients to fall asleep, then play for a full eight to nine hours during sleep.
4. The selected music is adapted for overnight listening in such a way as to avoid the risk of waking the patient with any crescendos or other spikes.

Examples of the general INRM technology are described in US9736603B2, US10587967B2, EP2729931B1 and WO2012168740A1.

Step 2) is described as follows:
Healthy EEG data is fed into MODA (Multiscale Oscillatory Dynamics Analysis - public domain) (Iatsenko (2015)) where a time-frequency analysis (tfa) is performed using the wavelet transform. This tfa is then split into three frequency bands; delta (0.8-3.5 Hz), theta/alpha (3.5-12 Hz) and gamma (12-40 Hz). Each of these bands are analysed using the ridge extraction protocol in MODA which extracts the amplitude and phase along a path in time frequency space connecting peak amplitudes in the band called a ridge. The amplitude and phase signals are then up-sampled to a sampling rate appropriate for musical signals (e.g. from 500 Hz to 44.5 kHz) by adding a fixed number of new samples along a straight line evenly connecting every pair of old samples. Each of the three pairs of up-sampled phase and amplitude data are then reconstructed into a higher frequency musical version of the ridge extracted from the EEG data by scaling the phase by 2 to the power of the desired number of octaves in a 'ridge reconstruction' equation $x \left(t\right) = A \left(t\right) sin \left(2oϕ\left(t\right)\right) + harmonics ,$ where x is the new musical ridge, A is the up-sampled amplitude, ϕ is the up-sampled phase and o is the number of octaves (e.g o = 6). Harmonics can be added by introducing more sine waves with phases scaled by 2o+1, o+2, etc and amplitudes scaled by some appropriate descending sequence (e.g 1, 2 , 1, 4, 1, 8, ...) to make each ridge more musical.

The result of this process is an audification featuring three ridges sounding simultaneously. This audification is then analysed by X-system, which categorises the audification based on its own internal parameters and uses them to search existing repertoire for music which is a close match to create the playlist.

Step 4) is described as follows:
After the X System curation, playlist tracks are downloaded and treated in the following sequence of post-processing:
- silence is trimmed from the front and back
- tracks are normalised to peak -0.1 dB
- tracks are cross-faded with approximately ten seconds at the beginning and end of each track
- filtering is applied to any frequencies (approximately 250 - 2000 Hz) which may appear 'harsh' or inordinately loud, due to the increased sensitivity of the ear to this part of the audio spectrum
- tracks are compressed with a large ratio and low threshold to ensure an even listening experience without dramatic changes in amplitude. This is done to mitigate the risk of waking the listener, which might otherwise happen at moments of wakefulness or light sleep between sleep cycles that occur naturally throughout the night.
- volume is set at maximum 40dB - with a decibel meter on the first night and physically marked on the speakers.

WHAT IS A POSSIBLE APPLICATION? an effective treatment for the 30% of epileptic patients who do not respond to medication.

WHY USE X-SYSTEM? no damaging side-effects and an alternative to surgery or vagus nerve stimulation.

HOW DOES IT WORK? In an example, proprietary algorithms and machine intelligence select the right music in the right order to entrain healthy patterns of brainwave activity.

WHAT IS DIFFERENT ABOUT X-SYSTEM?
- X-System's algorithms model how the brain reacts to music;
- it is radically different to traditional signal processing analysis; these algorithms predict the universal response of the primitive brain to patterns in music;
- Composers understand how they want their music to make you feel: X-System is the first technology to explain it
- This enables music to be selected automatically to have a specific therapeutic effect.
- Results can be validated biometrically by EEG data analysis, heart rate, heart rate variability, galvanic skin conductance or endocrine analysis.

### PILOT STUDY ON CHILDREN WITH RARE EPILEPSIES

We have conducted a pilot study in a leading Croatian national medical centre on three children with rare epilepsies.

Led by leading neurologists, the study used X-System technology to select music to reduce overnight brain rhythmic abnormalities and epileptic spikes (e.g. inter-ictal epileptiform discharges).

The hypothesis is that by influencing brainwave patterns the benefits will carry over into daytime activities, decreasing care and medication needs and improving behaviour problems.

The process involves taking overnight, medical grade EEG data, sonifying them and using X-System to select music from a diverse repertoire that may be expected to entrain healthy brain activity in subsequent night time listening.

### PRELIMINARY RESULTS:

An average reduction in epileptic spikes (e.g. inter-ictal epileptiform discharges) of 37%.

This compares with an average of 14.7% in three recent studies of the beneficial effect of an intuitive choice of music (Mozart, Bach and Haydn).

Significant increase in REM sleep, not seen in previous studies.

### POSSIBLE STEPS:

Automate the sonification of patients' EEG data and the selection/adjustment of sound recordings chosen for individual playlists.

Develop user interface and obtain Category IIa UK medical certification.

Secure licensed access to music catalogue.

### OTHER APPLICATIONS INCLUDE:

Treatment of general anxiety, panic disorder, Post-traumatic stress disorder (PTSD), sleep disorders, chronic pain and depression.

Treatment of pre-operative anxiety, mental state during specific medical procedures and post-operative pain management.

The management of severely psychotic patients in mental institutions.

The treatment of rare epilepsies and movement disorders which have not responded to available medications.

Mood management and general mental wellbeing.

### References

Baumgartner T, Lutz K, Schmidt CF, Jäncke L. The emotional power of music: how music enhances the feeling of affective pictures. Brain research. 2006; 1075(1):151-164.
Blood AJ, Zatorre RJ. Intensely pleasurable responses to music correlate with activity in brain regions implicated in reward and emotion. Proceedings of the national academy of sciences. 2001; 98(20):11818-11823.
Breitling D, Guenther W, Rondot P. Auditory perception of music measured by brain electrical activity mapping. Neuropsychologia. 1987; 25(5):765-774.
Eldar E, Ganor O, Admon R, Bleich A, Hendler T. Feeling the real world: limbic response to music depends on related content. Cerebral Cortex. 2007; 17(12):2828-2840.
Erlich N, Lipp OV, Slaughter V. Of hissing snakes and angry voices: human infants are differentially responsive to evolutionary fear-relevant sounds. Developmental science. 2013; 16(6):894-904.
Faust O, Acharya UR, Adeli H, Adeli A. Wavelet-based EEG processing for computer-aided seizure detection and epilepsy diagnosis. Seizure. 2015; 26:56-64. doi: https://doi.org/10.1016/j.seizure.2015.01.012.
Flanagan JL, Golden RM. Phase vocoder. The Bell System Technical Journal. 1966; 45(9):1493-1509. doi: 10.1002/j.1538-7305.1966.tb01706.x.
Frankland PW, Josselyn SA, Bradwejn J, Vaccarino FJ, Yeomans JS. Activation of amygdala cholecystokininB receptors potentiates the acoustic startle response in the rat. Journal of Neuroscience. 1997; 17(5):1838- 1847.
Griffiths TD, Büchel C, Frackowiak RS, Patterson RD. Analysis of temporal structure in sound by the human brain. Nature neuroscience. 1998; 1(5):422-427.
İnan Güler, Übeyli ED. Adaptive neuro-fuzzy inference system for classification of EEG signals using wavelet coefficients. Journal of Neuroscience Methods. 2005; 148(2):113-121. doi: https://doi.org/10.1016/j.jneumeth.2005.04.013.
Heldt SA, Falls WA. Destruction of the inferior colliculus disrupts the production and inhibition of fear conditioned to an acoustic stimulus. Behavioural brain research. 2003; 144(1-2):175-185.
Iatsenko D, Bernjak A, Stankovski T, Shiogai Y, Owen-Lynch P, Clarkson P, McClintock P, Stefanovska A. Evolution of cardiorespiratory interactions with age. Philosophical transactions Series A, Mathematical, physical, and engineering sciences. 2013 Aug; 371(1997). doi: 10.1098/rsta.2011.0622.
Iatsenko D, McClintock PVE, Stefanovska A. Extraction of instantaneous frequencies from ridges in time-frequency representations of signals. Signal Processing. 2016; 125:290-303. doi: https://doi.org/10.1016/j.sigpro.2016.01.024.
Iatsenko D, McClintock PVE, Stefanovska A. Linear and synchrosqueezed time-frequency representations revisited: Overview, standards of use, resolution, reconstruction, concentration, and algorithms. Digital Signal Processing. 2015; 42:1-26, doi: https://doi.org/10.1016/5.dsp.2015.03.004.
JORIS PX, SCHREINER CE, REES A. Neural Processing of Amplitude-Modulated Sounds. Physiological Reviews. 2004; 84(2):541-577, doi: 10.1152/physrev.00029.2003, pMID: 15044682.
Koelsch S, Fritz T, Schlaug G. Amygdala activity can be modulated by unexpected chord functions during music listening. Neuroreport. 2008; 19(18):1815-1819.
Koelsch S, Remppis A, Sammler D, Jentschke S, Mietchen D, Fritz T, Bonnemeier H, Siebel WA. A cardiac signature of emotionality. European Journal of Neuroscience. 2007; 26(11):3328-3338.
Laroche J, Dolson M. Improved phase vocoder time-scale modification of audio. IEEE Transactions on Speech and Audio Processing. 1999; 7(3):323-332. doi: 10.1109/89.759041.
Marsh RA, Fuzessery ZM, Grose CD, Wenstrup JJ. Projection to the inferior colliculus from the basal nucleus of the amygdala. Journal of Neuroscience. 2002; 22(23):10449-10460.
McDermott JH, Lehr AJ, Oxenham AJ. Individual differences reveal the basis of consonance. Current Biology. 2010; 20(11):1035-1041.
Menon V, Levitin DJ, Smith BK, Lembke A, Krasnow B, Glazer D, Glover GH, McAdams S. Neural correlates of timbre change in harmonic sounds. Neuroimage. 2002; 17(4):1742-1754.
Okawa H, Suefusa K, Tanaka T. Neural entrainment to auditory imagery of rhythms. Frontiers in human neuroscience. 2017; 11:493.
Osborne N. Towards a chronobiology of musical rhythm. Communicative musicality: Exploring the basis of human companionship. 2009; p. 545-564.
Panksepp J. Can anthropomorphic analyses of separation cries in other animals inform us about the emotional nature of social loss in humans? Comment on Blumberg and Sokoloff (2001).. 2003; .
Panksepp J. Affective neuroscience: The foundations of human and animal emotions. Oxford university press; 2004.
Penhune VB, Zatorre R, Feindel W. The role of auditory cortex in retention of rhythmic patterns as studied in patients with temporal lobe removals including Heschls gyrus. Neuropsychologia. 1999; 37(3):315-331.
Peretz I. Listen to the brain: A biological perspective on musical emotions.. 2001; .
Peretz I, AubéW, Armony J. Toward a neurobiology of musical emotions. Evol Emot Commun FromSoundsNonhum Mamm to Speech Music Man. 2010 01; p. 277-299. doi: 10.1093/acprof:oso/9780199583560.003.0017.
Peretz I, Kolinsky R. Boundaries of separability between melody and rhythm in music discrimination: A neuropsychological perspective. The Quarterly Journal of Experimental Psychology. 1993; 46(2):301-325.
Pérez-Riera AR, de Abreu LC, Barbosa-Barros R, Nikus KC, Baranchuk A. R-Peak Time: An Electrocardiographic Parameter with Multiple Clinical Applications. Annals of Noninvasive Electrocardiology. 2016; 21(1):10-19, doi: https://doi.org/10.1111/anec.12323.
Rafiee M, Istasy M, Valiante TA. Music in epilepsy: Predicting the effects of the unpredictable. Epilepsy & Behavior. 2021; 122:108164.
Ramos J, Corsi-cabrera M. Does Brain Electrical Activity React to Music? International Journal of Neuroscience. 1989; 47(3-4):351-357, doi: 10.3109/00207458908987449.
Sacks OW. Tales of Music and the Brain. Picador London, UK:; 2007.
Schneider P, Sluming V, Roberts N, Bleeck S, Rupp A. Structural, functional, and perceptual differences in Heschl's gyrus and musical instrument preference. Annals of the New York Academy of Sciences. 2005; 1060(1):387-394.
Sivaramakrishnan S, Sterbing-D'Angelo SJ, Filipovic B, D'Angelo WR, Oliver DL, Kuwada S. GABAA Synapses Shape Neuronal Responses to Sound Intensity in the Inferior Colliculus. Journal of Neuroscience. 2004; 24(21):5031-5043, doi: 10.1523/JNEUROSCI.0357-04.2004.
Stegemöller EL, Izbicki P, Hibbing P. The influence of moving with music on motor cortical activity. Neuroscience Letters. 2018; 683:27-32., doi: https://doi.org/10.1016/j.neulet.2018.06.030.
Stein MB, Simmons AN, Feinstein JS, Paulus MP. Increased amygdala and insula activation during emotion processing in anxiety-prone subjects. American Journal of Psychiatry. 2007; 164(2):318-327.
Ticcinelli V, Stankovski T, Iatsenko D, Bernjak A, Bradbury AE, Gallagher AR, Clarkson PBM, McClintock PVE, Stefanovska A. Coherence and Coupling Functions Reveal Microvascular Impairment in Treated Hypertension. Frontiers in Physiology. 2017; 8, doi:
   Unser M, Aldroubi A. A review of wavelets in biomedical applications. Proceedings of the IEEE. 1996; 84(4):626-638. doi: 10.1109/5.488704.
Warren JD, Uppenkamp S, Patterson RD, Griffiths TD. Separating pitch chroma and pitch height in the human brain. Proceedings of the National Academy of Sciences. 2003; 100(17):10038-10042.

### Note

It is to be understood that the above-referenced arrangements are only illustrative of the application for the principles of the present invention. Numerous modifications and alternative arrangements can be devised without departing from the scope of the present invention.

## Claims

1. A computer system, the computer system including music audio files including respective music file audio data, the computer system configured to:
(i) receive a file of electroencephalogram (EEG) data comprising EEG data of healthy brain behaviour of a human subject;
(ii) audify the EEG data of healthy brain behaviour of the human subject;
(iii) analyse the audified EEG data according to a neuro-physiological model of the principal areas and networks of the human brain involved in processing music, to produce analysis data of the audified EEG data;
(iv) analyse the music file audio data according to the neuro-physiological model of the principal areas and networks of the human brain involved in processing music, to produce analysis data of the music file audio data;
(v) compare the analysis data of the audified EEG data with the analysis data of the music file audio data, to match the analysis data of the audified EEG data with matched analysis data of the music file audio data, to produce a playlist of matched music audio files corresponding to the matched music file audio data, wherein the matched music audio files are suitable to entrain healthy brain behaviour in the human subject; and
(vi) store the playlist of matched music audio files.

2. The computer system of Claim 1, the computer system (e.g. an audio playback device) including a speaker or headphones or a sound reproduction device, the computer system further configured to play the playlist of matched music audio files to the human subject, including outputting played matched music audio files to the speaker or to the headphones or to the sound reproduction device.

3. The computer system of any previous Claim, wherein audifying the EEG data comprises:
(a) sampling the EEG data at a rate in the range of 100 Hz to 2.5kHz;
(b) extracting sinusoidal waves with time varying frequency from the sampled EEG data from at least three frequency bands in the range of 0.8 to 40 Hz;
(c)upsampling the extracted sinusoidal waves with time varying frequency to a rate in the range of 10 kHz to 100kHz;
(d) constructing signals for the upsampled extracted sinusoidal waves with time varying frequency by scaling the upsampled extracted sinusoidal waves with time varying frequency in the range of 5 to 9 octaves, to provide audified EEG data.

4. The computer system of Claim 3, wherein in (a) the EEG data is sampled at a rate in the range of 200 Hz to 1 kHz; or wherein in (a) the EEG data is sampled at a rate of 500 Hz; or wherein (a) includes subtracting a 3rd order polynomial fit to remove trends which can skew time-frequency representation (TFR) and then the signal is low-pass filtered at the Nyquist frequency.

5. The computer system of any of Claims 3 to 4, wherein in (b) there are three frequency bands; or wherein in (b) there are three frequency bands which are 0.8-3.5Hz, 3.5-12Hz, and 12-40Hz.

6. The computer system of any of Claims 3 to 5, wherein in (b) the analysis is performed using time-frequency representations (TFRs); or wherein in (b) ridge extraction is used; or wherein in (b) extracting sinusoidal waves with time varying frequency from the sampled EEG data is performed using Multiscale oscillatory dynamics analysis (MODA).

7. The computer system of any of Claims 3 to 6, wherein in (c ) the extracted sinusoidal waves with time varying frequency are upsampled to a rate in the range from 20 kHz to 80 kHz; or wherein in (c ) the extracted sinusoidal waves with time varying frequency are upsampled to a rate of 44.5 kHz.

8. The computer system of any of Claims 3 to 7, wherein in (d), the upsampled extracted sinusoidal waves with time varying frequency are scaled by 5 octaves, or by 6 octaves, or by 7 octaves, or by 8 octaves, or by 9 octaves; or wherein in (d), a factor of 2 to the power of the number of octaves to be scaled by is inserted into a ridge reconstruction equation, e.g. Eq. (2).

9. The computer system of any previous Claim, wherein in parts (iii) and (iv), the analysis is performed by analysing for volume, turbulence, sharpness, rhythmicity, and harmonicity H.

10. The computer system of Claim 9, wherein rhythmicity analysis includes detecting power, salience and density of periodic spectral turbulence.

11. The computer system of any previous Claim, wherein the file of electroencephalogram (EEG) data comprising EEG data of healthy brain behaviour of a human subject is in the range of 1 minute to 100 minutes in duration; or wherein the file of electroencephalogram (EEG) data comprising EEG data of healthy brain behaviour of a human subject is in the range of 3 minutes to 30 minutes in duration; or wherein the file of electroencephalogram (EEG) data comprising EEG data of healthy brain behaviour of a human subject is 10 minutes in duration.

12. The computer system of any previous Claim, wherein the file of electroencephalogram (EEG) data comprising EEG data of healthy brain behaviour of a human subject begins with EEG data corresponding to wakefulness, then continues with EEG data corresponding to sleep.

13. The computer system of any of Claims 1 to 11, wherein the file of electroencephalogram (EEG) data comprising EEG data of healthy brain behaviour of a human subject includes only EEG data corresponding to sleep.

14. The computer system of any previous Claim, wherein the playlist is 1 to 12 hours in duration; or wherein the playlist is 6 to 10 hours in duration; or wherein the playlist is 9 hours in duration.

15. A computer-implemented method for generating a playlist of music audio files suitable to provide healthy brain behaviour in a human subject, the method including the steps of:
(i) receiving a file of electroencephalogram (EEG) data comprising EEG data of healthy brain behaviour of a human subject;
(ii) audifying the EEG data of healthy brain behaviour of the human subject;
(iii) analysing the audified EEG data according to a neuro-physiological model of the principal areas and networks of the human brain involved in processing music, to produce analysis data of the audified EEG data;
(iv) accessing music audio files including respective music file audio data;
(v) analysing the music file audio data of the music audio files according to the neuro-physiological model of the principal areas and networks of the human brain involved in processing music, to produce analysis data of the music file audio data;
(vi) comparing the analysis data of the audified EEG data with the analysis data of the music file audio data, to match the analysis data of the audified EEG data with matched analysis data of the music file audio data, to produce a playlist of matched music audio files corresponding to the matched music file audio data, wherein the matched music audio files are suitable to entrain healthy brain behaviour in the human subject; and
(vii) storing the playlist of matched music audio files.

## Patentansprüche

1. Computersystem, wobei das Computersystem Musikaudiodateien beinhaltet, die jeweilige Musikdatei-Audiodaten beinhalten, wobei das Computersystem zu Folgendem konfiguriert ist:
(i) Empfangen einer Datei mit Elektroenzephalogrammdaten (EEG-Daten), die EEG-Daten eines gesunden Gehirnverhaltens eines menschlichen Probanden umfassen;
(ii) Audifizieren der EEG-Daten eines gesunden Gehirnverhaltens des menschlichen Probanden;
(iii) Analysieren der audifizierten EEG-Daten gemäß einem neuro-physiologischen Modell der Hauptbereiche und Netze des menschlichen Gehirns, die in ein Verarbeiten von Musik involviert sind, dazu, Analysedaten der audifizierten EEG-Daten zu produzieren;
(iv) Analysieren der Musikdatei-Audiodaten gemäß dem neuro-physiologischen Modell der Hauptbereiche und Netze des menschlichen Gehirns, die in ein Verarbeiten von Musik involviert sind, dazu, Analysedaten der Musikdatei-Audiodaten zu produzieren;
(v) Vergleichen der Analysedaten der audifizierten EEG-Daten mit den Analysedaten der Musikdatei-Audiodaten, um die Analysedaten der audifizierten EEG-Daten mit abgestimmten Analysedaten der Musikdatei-Audiodaten abzustimmen, um eine Wiedergabeliste abgestimmter Musikaudiodateien zu produzieren, die den abgestimmten Musikdatei-Audiodaten entsprechen, wobei die abgestimmten Musikaudiodateien geeignet sind, ein gesundes Gehirnverhalten bei dem menschlichen Probanden anzuregen; und
(vi) Speichern der Wiedergabeliste abgestimmter Musikaudiodateien.

2. Computersystem nach Anspruch 1, wobei das Computersystem (z. B. ein Audiowiedergabegerät) einen Lautsprecher oder Kopfhörer oder ein Tonwiedergabegerät beinhaltet, wobei das Computersystem ferner dazu konfiguriert ist, die Wiedergabeliste abgestimmter Musikaudiodateien für den menschlichen Probanden abzuspielen, was Ausgeben abgespielter abgestimmter Musikaudiodateien auf dem Lautsprecher oder dem Kopfhörer oder dem Tonwiedergabegerät beinhaltet.

3. Computersystem nach einem der vorhergehenden Ansprüche, wobei das Audifizieren der EEG-Daten Folgendes umfasst:
(a) Abtasten der EEG-Daten mit einer Rate im Bereich von 100 Hz bis 2,5 kHz;
(b) Extrahieren von Sinuswellen mit zeitlich variierender Frequenz aus den abgetasteten EEG-Daten aus mindestens drei Frequenzbändern im Bereich von 0,8 bis 40 Hz;
(c) Hochabtasten der extrahierten Sinuswellen mit zeitlich variierender Frequenz auf eine Rate im Bereich von 10 kHz bis 100 kHz;
(d) Konstruieren von Signalen für die hochabgetasteten extrahierten Sinuswellen mit zeitlich variierender Frequenz durch Skalieren der hochabgetasteten extrahierten Sinuswellen mit zeitlich variierender Frequenz im Bereich von 5 bis 9 Oktaven, um audifizierte EEG-Daten bereitzustellen.

4. Computersystem nach Anspruch 3, wobei in (a) die EEG-Daten mit einer Rate im Bereich von 200 Hz bis 1 kHz abgetastet werden; oder wobei in (a) die EEG-Daten mit einer Rate von 500 Hz abgetastet werden; oder wobei (a) das Subtrahieren einer Polynomanpassung dritter Ordnung dazu beinhaltet, Trends zu entfernen, die die Zeit-Frequenz-Darstellung (TFR) verzerren können, und das Signal anschließend bei der Nyquist-Frequenz tiefpassgefiltert wird.

5. Computersystem nach einem der Ansprüche 3 bis 4, wobei es in (b) drei Frequenzbänder gibt; oder wobei es in (b) drei Frequenzbänder gibt, die 0,8-3,5 Hz, 3,5-12 Hz und 12-40 Hz betragen.

6. Computersystem nach einem der Ansprüche 3 bis 5, wobei in (b) die Analyse unter Verwendung von Zeit-Frequenz-Darstellungen (TFRs) durchgeführt wird; oder wobei in (b) die Ridge-Extraktion verwendet wird; oder wobei in (b) das Extrahieren von Sinuswellen mit zeitlich variierender Frequenz aus den abgetasteten EEG-Daten unter Verwendung einer Analyse der multiskaligen Schwingungsdynamik (MODA) durchgeführt wird.

7. Computersystem nach einem der Ansprüche 3 bis 6, wobei in (c) die extrahierten Sinuswellen mit zeitlich variierender Frequenz auf eine Rate im Bereich von 20 kHz bis 80 kHz hochabgetastet sind; oder wobei in (c) die extrahierten Sinuswellen mit zeitlich variierender Frequenz auf eine Rate von 44,5 kHz hochabgetastet sind.

8. Computersystem nach einem der Ansprüche 3 bis 7, wobei in (d) die hochabgetasteten extrahierten Sinuswellen mit zeitlich variierender Frequenz um 5 Oktaven oder um 6 Oktaven oder um 7 Oktaven oder um 8 Oktaven oder um 9 Oktaven skaliert sind; oder wobei in (d) ein Faktor von 2 hoch der Anzahl der zu skalierenden Oktaven in eine Ridge-Rekonstruktionsgleichung eingesetzt wird, z. B. Gleichung (2).

9. Computersystem nach einem der vorhergehenden Ansprüche, wobei in den Teilen (iii) und (iv) die Analyse durch Analysieren auf Volumen, Turbulenz, Schärfe, Rhythmik und Harmonie H durchgeführt wird.

10. Computersystem nach Anspruch 9, wobei die Rhythmusanalyse Erkennen von Leistung, Salienz und Dichte periodischer spektraler Turbulenzen beinhaltet.

11. Computersystem nach einem der vorhergehenden Ansprüche, wobei die Dauer der Datei mit Elektroenzephalogrammdaten (EEG-Daten), die EEG-Daten eines gesunden Gehirnverhaltens eines menschlichen Probanden umfassen, im Bereich von 1 Minute bis 100 Minuten liegt; oder wobei die Dauer der Datei mit Elektroenzephalogrammdaten (EEG-Daten), die EEG-Daten eines gesunden Gehirnverhaltens eines menschlichen Probanden umfassen, im Bereich von 3 Minuten bis 30 Minuten liegt; oder wobei die Datei der Elektroenzephalogrammdaten (EEG-Daten), die EEG-Daten eines gesunden Gehirnverhaltens eines menschlichen Probanden umfassen, eine Dauer von 10 Minuten hat.

12. Computersystem nach einem der vorhergehenden Ansprüche, wobei die Datei mit Elektroenzephalogrammdaten (EEG-Daten), die EEG-Daten eines gesunden Gehirnverhaltens eines menschlichen Probanden umfassen, mit EEG-Daten beginnt, die Wachheit entsprechen, und dann mit EEG-Daten fortfährt, die Schlaf entsprechen.

13. Computersystem nach einem der Ansprüche 1 bis 11, wobei die Datei mit Elektroenzephalogrammdaten (EEG-Daten), die EEG-Daten eines gesunden Gehirnverhaltens eines menschlichen Probanden umfassen, nur EEG-Daten beinhaltet, die Schlaf entsprechen.

14. Computersystem nach einem der vorhergehenden Ansprüche, wobei die Wiedergabeliste eine Dauer von 1 bis 12 Stunden hat; oder wobei die Wiedergabeliste eine Dauer von 6 bis 10 Stunden hat; oder wobei die Wiedergabeliste eine Dauer von 9 Stunden hat.

15. Computerimplementiertes Verfahren zum Erzeugen einer Wiedergabeliste mit Musikaudiodateien, die geeignet ist, für ein gesundes Gehirnverhalten bei einem menschlichen Probanden zu sorgen, wobei das Verfahren die folgenden Schritte beinhaltet:
(i) Empfangen einer Datei mit Elektroenzephalogrammdaten (EEG-Daten), die EEG-Daten eines gesunden Gehirnverhaltens eines menschlichen Probanden umfassen;
(ii) Audifizieren der EEG-Daten eines gesunden Gehirnverhaltens des menschlichen Probanden;
(iii) Analysieren der audifizierten EEG-Daten gemäß einem neuro-physiologischen Modell der Hauptbereiche und Netze des menschlichen Gehirns, die in ein Verarbeiten von Musik involviert sind, dazu, Analysedaten der audifizierten EEG-Daten zu produzieren;
(iv) Zugreifen auf Musikaudiodateien, die jeweilige Musikdatei-Audiodaten beinhalten;
(v) Analysieren der Musikdatei-Audiodaten der Musikaudiodateien gemäß dem neuro-physiologischen Modell der Hauptbereiche und Netze des menschlichen Gehirns, die in ein Verarbeiten von Musik involviert sind, dazu, Analysedaten der Musikdatei-Audiodaten zu produzieren;
(vi) Vergleichen der Analysedaten der audifizierten EEG-Daten mit den Analysedaten der Musikdatei-Audiodaten, um die Analysedaten der audifizierten EEG-Daten mit abgestimmten Analysedaten der Musikdatei-Audiodaten abzustimmen, um eine Wiedergabeliste abgestimmter Musikaudiodateien zu produzieren, die den abgestimmten Musikdatei-Audiodaten entsprechen, wobei die abgestimmten Musikaudiodateien geeignet sind, um ein gesundes Gehirnverhalten bei dem menschlichen Probanden anzuregen; und
(vii) Speichern der Wiedergabeliste abgestimmter Musikaudiodateien.

## Revendications

1. Système informatique, le système informatique incluant des fichiers audio de musique incluant des données audio de fichier de musique respectives, le système informatique étant configuré pour :
(i) recevoir un fichier de données d'électroencéphalogramme (EEG) comprenant des données d'EEG de comportement cérébral sain d'un sujet humain ;
(ii) sonifier les données d'EEG de comportement cérébral sain du sujet humain ;
(iii) analyser les données d'EEG sonifiées selon un modèle neurophysiologique des zones et réseaux principaux du cerveau humain impliqués dans le traitement de la musique, pour produire des données d'analyse des données d'EEG sonifiées ;
(iv) analyser les données audio de fichier de musique selon le modèle neurophysiologique des zones et réseaux principaux du cerveau humain impliqués dans le traitement de la musique, pour produire des données d'analyse des données audio de fichier de musique ;
(v) comparer les données d'analyse des données d'EEG sonifiées avec les données d'analyse des données audio de fichier de musique, pour faire correspondre les données d'analyse des données d'EEG sonifiées avec des données d'analyse mises en correspondance des données audio de fichier de musique, pour produire une liste de lecture de fichiers audio de musique mis en correspondance correspondant aux données audio de fichier de musique mises en correspondance, dans lequel les fichiers audio de musique mis en correspondance sont appropriés pour entraîner un comportement cérébral sain chez le sujet humain ; et
(vi) stocker la liste de lecture de fichiers audio de musique mis en correspondance.

2. Système informatique selon la revendication 1, le système informatique (par exemple un dispositif de lecture audio) incluant un haut-parleur ou des écouteurs ou un dispositif de reproduction sonore, le système informatique étant en outre configuré pour lire la liste de lecture de fichiers audio de musique mis en correspondance au sujet humain, incluant la délivrance en sortie de fichiers audio de musique mis en correspondance lus vers le haut-parleur ou vers les écouteurs ou vers le dispositif de reproduction sonore.

3. Système informatique selon l'une quelconque des revendications précédentes, dans lequel la sonification des données d'EEG comprend :
(a) l'échantillonnage des données d'EEG à une fréquence dans la plage de 100 Hz à 2.5 kHz ;
(b) l'extraction d'ondes sinusoïdales avec une fréquence variant dans le temps à partir des données d'EEG échantillonnées provenant d'au moins trois bandes de fréquences dans la plage de 0.8 à 40 Hz ;
(c) le suréchantillonnage des ondes sinusoïdales extraites avec une fréquence variant dans le temps à une fréquence dans la plage de 10 kHz à 100 kHz ;
(d) la construction de signaux pour les ondes sinusoïdales extraites suréchantillonnées avec une fréquence variant dans le temps par la mise à l'échelle des ondes sinusoïdales extraites suréchantillonnées avec une fréquence variant dans le temps dans la plage de 5 à 9 octaves, pour fournir des données d'EEG sonifiées.

4. Système informatique selon la revendication 3, dans lequel dans (a) les données d'EEG sont échantillonnées à une fréquence dans la plage de 200 Hz à 1 kHz ; ou dans lequel dans (a) les données d'EEG sont échantillonnées à une fréquence de 500 Hz ; ou dans lequel (a) inclut la soustraction d'un ajustement polynomial de 3e ordre pour supprimer des tendances qui peuvent biaiser une représentation temps-fréquence (RTF) et ensuite le signal est soumis à un filtrage passe-bas à la fréquence de Nyquist.

5. Système informatique selon l'une quelconque des revendications 3 à 4, dans lequel dans (b) il y a trois bandes de fréquences ; ou dans lequel dans (b) il y a trois bandes de fréquences qui sont 0.8 à 3.5 Hz, 3.5 à 12 Hz, et 12 à 40 Hz.

6. Système informatique selon l'une quelconque des revendications 3 à 5, dans lequel dans (b) l'analyse est réalisée en utilisant des représentations temps-fréquence (RTF) ; ou dans lequel dans (b) une extraction de crête est utilisée ; ou dans lequel dans (b) l'extraction d'ondes sinusoïdales avec une fréquence variant dans le temps à partir des données d'EEG échantillonnées est réalisée en utilisant une analyse de dynamique oscillatoire multi-échelle (ADOME).

7. Système informatique selon l'une quelconque des revendications 3 à 6, dans lequel dans (c) les ondes sinusoïdales extraites avec une fréquence variant dans le temps sont suréchantillonnées à une fréquence dans la plage allant de 20 kHz à 80 kHz ; ou dans lequel dans (c) les ondes sinusoïdales extraites avec une fréquence variant dans le temps sont suréchantillonnées à une fréquence de 44.5 kHz.

8. Système informatique selon l'une quelconque des revendications 3 à 7, dans lequel dans (d), les ondes sinusoïdales extraites suréchantillonnées avec une fréquence variant dans le temps sont mises à l'échelle par 5 octaves, ou par 6 octaves, ou par 7 octaves, ou par 8 octaves, ou par 9 octaves ; ou dans lequel dans (d), un facteur de 2 à la puissance du nombre d'octaves par lequel mettre à l'échelle est inséré dans une équation de reconstruction de crête, par exemple l'Éq. (2).

9. Système informatique selon l'une quelconque des revendications précédentes, dans lequel dans les parties (iii) et (iv), l'analyse est réalisée par analyse du volume, de la turbulence, de la netteté, de la rythmicité, et de l'harmonicité H.

10. Système informatique selon la revendication 9, dans lequel l'analyse de rythmicité inclut la détection de la puissance, de la saillance et de la densité d'une turbulence spectrale périodique.

11. Système informatique selon l'une quelconque des revendications précédentes, dans lequel le fichier de données d'électroencéphalogramme (EEG) comprenant des données d'EEG de comportement cérébral sain d'un sujet humain a une durée dans la plage de 1 minute à 100 minutes ; ou dans lequel le fichier de données d'électroencéphalogramme (EEG) comprenant des données d'EEG de comportement cérébral sain d'un sujet humain a une durée dans la plage de 3 minutes à 30 minutes ; ou dans lequel le fichier de données d'électroencéphalogramme (EEG) comprenant des données d'EEG de comportement cérébral sain d'un sujet humain a une durée de 10 minutes.

12. Système informatique selon l'une quelconque des revendications précédentes, dans lequel le fichier de données d'électroencéphalogramme (EEG) comprenant des données d'EEG de comportement cérébral sain d'un sujet humain commence par des données d'EEG correspondant à l'éveil, puis continue avec des données d'EEG correspondant au sommeil.

13. Système informatique selon l'une quelconque des revendications 1 à 11, dans lequel le fichier de données d'électroencéphalogramme (EEG) comprenant des données d'EEG de comportement cérébral sain d'un sujet humain inclut uniquement des données d'EEG correspondant au sommeil.

14. Système informatique selon l'une quelconque des revendications précédentes, dans lequel la liste de lecture a une durée de 1 à 12 heures ; ou dans lequel la liste de lecture a une durée de 6 à 10 heures ; ou dans lequel la liste de lecture a une durée de 9 heures.

15. Procédé mis en œuvre par ordinateur pour générer une liste de lecture de fichiers audio de musique appropriés pour induire un comportement cérébral sain chez un sujet humain, le procédé incluant les étapes consistant à :
(i) recevoir un fichier de données d'électroencéphalogramme (EEG) comprenant des données d'EEG de comportement cérébral sain d'un sujet humain ;
(ii) sonifier les données d'EEG de comportement cérébral sain du sujet humain ;
(iii) analyser les données d'EEG sonifiées selon un modèle neurophysiologique des zones et réseaux principaux du cerveau humain impliqués dans le traitement de la musique, pour produire des données d'analyse des données d'EEG sonifiées ;
(iv) accéder à des fichiers audio de musique incluant des données audio de fichier de musique respectives ;
(v) analyser les données audio de fichier de musique des fichiers audio de musique selon le modèle neurophysiologique des zones et réseaux principaux du cerveau humain impliqués dans le traitement de la musique, pour produire des données d'analyse des données audio de fichier de musique ;
(vi) comparer les données d'analyse des données d'EEG sonifiées avec les données d'analyse des données audio de fichier de musique, pour faire correspondre les données d'analyse des données d'EEG sonifiées avec des données d'analyse mises en correspondance des données audio de fichier de musique, pour produire une liste de lecture de fichiers audio de musique mis en correspondance correspondant aux données audio de fichier de musique mises en correspondance, dans lequel les fichiers audio de musique mis en correspondance sont appropriés pour entraîner un comportement cérébral sain chez le sujet humain ; et
(vii) stocker la liste de lecture de fichiers audio de musique mis en correspondance.
